# EUROPEAN PATENT APPLICATION

(11) **EP 2 474 835 A2**
(43) Date of publication of application: **11.07.2012**
(21) Application number: 12150293.4
(22) Date of filing: 05.01.2012
(51) Int. Cl.: G01S 7/52

(54) **Diagnosis system, medical image system, and method of displaying diagnosis image**

(30) Priority: 06.01.2011 KR 20110001552
(71) Applicant: Samsung Electronics Co., Ltd., Suwon-si, Gyeonggi-do 443-742 (KR); Industrie University Cooperation Foundation Sogang University, Seoul 121-742 (KR)
(72) Inventor: Kim, Bae-hyung, Yongin-si, Gyeonggi-do (KR); Chang, Jin-ho, Bucheon-si, Gyeonggi-do (KR); Song, Tai-kyong, Seoul (KR); Cho, Kyung-il, Yongin-si, Gyeonggi-do (KR); Kim, Dong-wook, Yongin-si, Gyeonggi-do (KR); Song, Jong-keun, Yongin-si, Gyeonggi-do (KR); Yoo, Yang-mo, Gimpo-si, Gyeonggi-do (KR)
(74) Representative: Grünecker, Kinkeldey, Stockmair & Schwanhäusser

(57) **Abstract**

A diagnosis system, a medical image system, and a method of displaying a diagnosis image are provided. The diagnosis system includes a probe (100) including a plurality of sub-arrays (61,62,63), each of the sub-arrays including at least one line of array transducers, the array transducers being configured to transmit and receive signals to and from a subject being diagnosed, and a hybrid beamformer configured to perform analog beamforming (220) in a direction in which the sub-arrays are arranged, and perform digital beamforming (241) in a direction perpendicular to the direction in which the sub-arrays are arranged.

## Description

### BACKGROUND

### 1. Field

The following description relates to a diagnosis system, a medical image system, and a method of displaying a diagnosis image.

### 2. Description of the Related Art

Medical ultrasonic image apparatuses are not harmful to the human body, and provide anatomic and functional information regarding internal body parts in real time. Medical ultrasonic image apparatuses may use 1-dimensional (1D) array transducers to provide 2-dimensional (2D) plane images of internal body parts. Furthermore, medical ultrasonic image apparatuses may provide 3-dimensional (3D) images of internal body parts by obtaining volume information of the internal body parts while mechanically moving 1-dimensional (1D) array transducers.

### SUMMARY

In one general aspect, there is provided a diagnosis system, including a probe including a plurality of sub-arrays, each of the sub-arrays including at least one line of array transducers, the array transducers being configured to transmit and receive signals to and from a subject being diagnosed, and a hybrid beamformer configured to perform analog beamforming in a direction in which the sub-arrays are arranged, and perform digital beamforming in a direction perpendicular to the direction in which the sub-arrays are arranged.

The general aspect of the diagnosis system may further provide that the hybrid beamformer includes a control unit configured to calculate time delay values for the digital beamforming for each of the sub-arrays, and control transducers included in each of the sub-arrays to transmit and receive the signals to and from the subject according to the time delay values calculated for each of the sub-arrays.

The general aspect of the diagnosis system may further provide that the control unit is further configured to calculate time delay values for the analog beamforming for each of transducers included in one of the sub-arrays, wherein the calculated time delay values for transducers being disposed in the same position in the direction perpendicular to the direction in which the sub-arrays are arranged, are the same, and control the transducers to transmit and receive the signals to and from the subject according to the calculated time delay values for the analog beamforming.

The general aspect of the diagnosis system may further provide that the hybrid beamformer includes an analog beamformer configured to combine the signals received by the transducers included in the sub-arrays according to the time delay values for the analog beamforming, and generate a plurality of analog signals for each of the sub-arrays.

The general aspect of the diagnosis system may further provide that the analog beamformer is further configured to steer the signals transmitted from the probe to the subject in the direction in which the sub-arrays are arranged.

The general aspect of the diagnosis system may further provide that the hybrid beamformer further includes an analog beamformer configured to combine the signals received by the transducers included in the sub-arrays according to the time delay values for analog beamforming, and generate a plurality of analog signals for each of the sub-arrays, an analog-digital converter configured to convert the generated analog signals into digital signals, and a digital beamformer configured to combine the converted digital signals according to the time delay values for the digital beamforming calculated for each of the sub-arrays.

The general aspect of the diagnosis system may further provide that the hybrid beamformer includes an analog beamformer configured to steer the signals transmitted from the transducers to the subject in an elevation direction when each of the sub-arrays includes a line of all transducers arranged in the elevation direction.

The general aspect of the diagnosis system may further provide that the hybrid beamformer includes an analog beamformer configured to steer the signals transmitted from the transducers to the subject in a lateral direction when each of the sub-arrays includes a line of all transducers arranged in the lateral direction.

In another aspect, there is provided a medical image system, including a probe including a plurality of sub-arrays, each of the sub-arrays including at least one line of array transducers, the array transducers being configured to transmit and receive signals to and from a subject, a hybrid beamformer configured to form a reception beam by performing analog beamforming in a direction in which the sub-arrays are arranged and performing digital beamforming in a direction perpendicular to the direction in which the sub-arrays are arranged, and a diagnosis image generating unit configured to generate a diagnosis image for the subject by using the formed reception beam.

The general aspect of the medical image system may further provide that the hybrid beamformer includes an analog beamformer configured to perform the analog beamforming, and steer signals transmitted from the probe to the subject in the direction in which the sub-arrays are arranged.

The general aspect of the medical image system may further provide that the hybrid beamformer includes a control unit configured to calculate time delay values for the digital beamforming for each of the sub-arrays, and control the transducers to transmit and receive the signals to and from the subject according to the time delay values calculated for each of the sub-arrays.

The general aspect of the medical image system may further provide that the control unit is further configured to calculate time delay values for the analog beamforming for each of the transducers included in one of the sub-arrays, wherein the calculated time delay values for transducers being disposed in the same position in the direction perpendicular to the direction in which the sub-arrays are arranged, are the same, and control the transducers to transmit and receive the signals to and from the subject according to the calculated time delay values.

The general aspect of the medical image system may further provide a diagnosis image display unit configured to display the generated diagnosis image.

In another aspect, there is provided a method of displaying a diagnosis image, the method including calculating time delay values for digital beamforming for each of a plurality of sub-arrays included in a probe and time delay values for analog beamforming for each of a plurality of transducers included in one of the sub-arrays, transmitting signals from the transducers included in the sub-arrays to a subject according to the calculated time delay values, combining the signals received by the transducers included in the sub-arrays according to the calculated time delay values for the analog beamforming, generating a plurality of analog signals for each of the sub-arrays, converting the analog signals into a plurality of digital signals, combining the digital signals according to the time delay values for the digital beamforming, and displaying a diagnosis image generated by using a result of the combining of the digital signals. Each of the sub-arrays comprises at least one line of the array transducers.

The general aspect of the method may further provide that the transmitting of the signals includes steering the signals in a direction in which the sub-arrays are arranged.

The general aspect of the method may further provide that each of the sub-arrays includes a line of all transducers arranged in an elevation direction or in a lateral direction.

The general aspect of the method may further provide that the transmitting of the signals includes steering the signals in an elevation direction when each of the sub-arrays comprises a line of all transducers arranged in the elevation direction,.

The general aspect of the method may further provide that the transmitting of the signals includes steering the signals in a lateral direction when each of the sub-arrays comprises a line of all transducers arranged in the lateral direction.

The general aspect of the method may further provide that the transducers have the same time delay value calculated for the analog beamforming, the transducers being included in one of the sub-arrays and disposed in the same position in the direction perpendicular to the direction in which the sub-arrays are arranged.

In another aspect, there is provided a non-transitory computer-readable recording medium having stored thereon instructions, wherein execution of the instructions by one or more processors of a computer system causes the one or more processors to perform the method of displaying the diagnosis image.

Other features and aspects may be apparent from the following detailed description, the drawings, and the claims.

### BRIEF DESCRIPTION OF THE DRAWINGS

FIG 1 illustrates an example of an environment in which a diagnosis system is used.
FIG 2 is a block diagram illustrating an example of a diagnosis system.
FIGS. 3A through 3D illustrate examples of sub-arrays of a probe of a diagnosis system.
FIG. 4 illustrates an example of a method of calculating time delay values for digital beamforming for each sub-array in a control unit of a hybrid beamformer of a diagnosis system.
FIGS. 5A and 5B illustrate examples of elevation and lateral directions in sub-arrays of a probe of a diagnosis system.
FIG 6 illustrates an example of a transmission beamforming operation of a diagnosis system.
FIG. 7 illustrates an example of a reception beamforming operation of a diagnosis system.
FIG. 8 is a block diagram illustrating an example of a medical image system.
FIG. 9 is a flowchart illustrating an example of a method of displaying a diagnosis image.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following detailed description is provided to assist the reader in gaining a comprehensive understanding of the methods and/or systems described herein. Accordingly, various changes, modifications, and equivalents of the systems and/or methods described herein will be suggested to those of ordinary skill in the art. Also, descriptions of well-known functions and constructions may be omitted for increased clarity and conciseness.

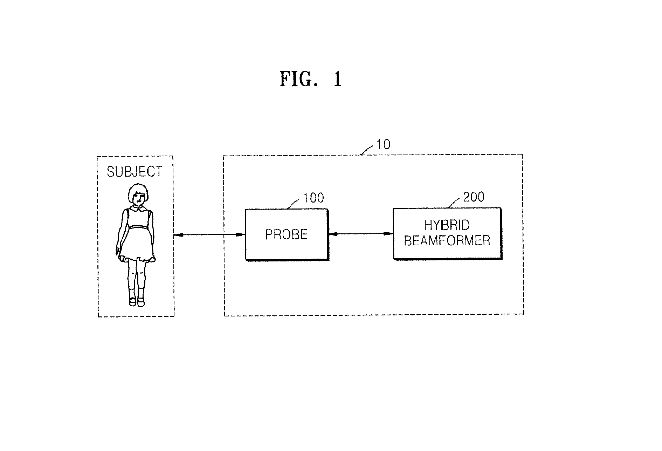

FIG. 1 illustrates an example of an environment in which a diagnosis system 10 is used. Referring to FIG 1, the diagnosis system 10 includes a probe 100 and a hybrid beamformer 200; however, the diagnosis system 10 is not limited thereto.

Although the probe 100 and the hybrid beamformer 200 are separated from each other in FIG. 1, the diagnosis system 10 is not limited thereto. The hybrid beamformer 200 may be combined with the probe 100.

The probe 100 of the diagnosis system 10 transmits and receives a signal to and from a subject. The hybrid beamformer 200 of the diagnosis system 10 forms a transmission beam, generates a signal that is converted and is to be transmitted to the subject by the probe 100, combines signals received by the probe 100, and forms a reception beam.

For example, the probe 100 of the diagnosis system 10 includes sub-arrays. Each of the sub-arrays include at least one line of array transducers that transmit and receive the signal to and from the subject. The hybrid beamformer 200 performs analog beamforming in a direction in which the sub-arrays are arranged, and performs digital beamforming in a direction perpendicular to the direction in which the sub-arrays are arranged. In this regard, the direction in which the sub-arrays are arranged may be an elevation direction or a lateral direction, and, thus, the direction perpendicular to the direction in which the sub-arrays are arranged may be the lateral direction or the elevation direction.

Therefore, the diagnosis system 10 may output a signal indicating information regarding the subject. The signal may be generated as a diagnosis image and may be displayed to a user. In this regard, although the diagnosis image of the diagnosis system 10 may be a 3-dimensional (3D) image, the diagnosis image is not limited thereto.

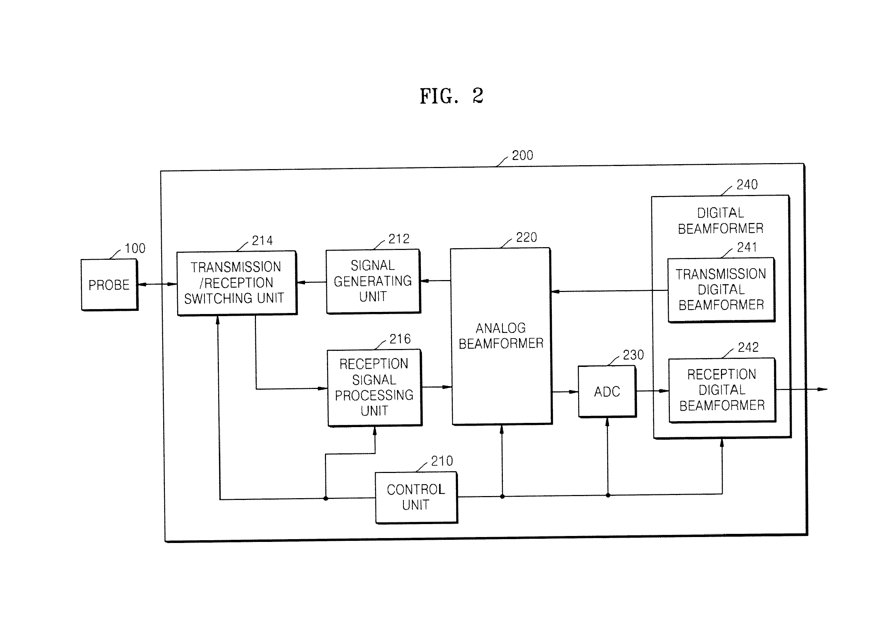

FIG. 2 is a block diagram illustrating an example of the diagnosis system 10. Referring to FIG 2, the diagnosis system 10 includes the probe 100 and the hybrid beamformer 200. The hybrid beamformer 200 includes a control unit 210, a signal generating unit 212, a transmission/reception switching unit 214, a reception signal processing unit 216, an analog beamformer 220, an analog-digital converter (ADC) 230, and a digital beamformer 240. The digital beamformer 240 includes a transmission digital beamformer 241 and a reception digital beamformer 242.

FIG. 2 illustrates examples of the elements of the diagnosis system 10. Accordingly, it would be understood by one of ordinary skill in the art that the diagnosis system 10 may further include general-purpose elements other than the elements illustrated in FIG. 2.

Furthermore, the control unit 210, the reception signal processing unit 216, the analog beamformer 220, the ADC 230, and the digital beamformer 240 of the diagnosis system 10 of FIG. 2 may be one processor or a plurality of processors. The processor may include an array of logic gates, or a combination of a general-purpose micro-processor and a memory in which a program that may be executed in the general-purpose micro-processor is stored. It would be understood by one of ordinary skill in the art that the processor may include a different type of hardware.

The probe 100 transmits and receives a signal to and from the subject. In this regard, although the signal may be an ultrasonic signal, the signal is not limited thereto. Accordingly, the probe 100 includes sub-arrays, each of the sub-arrays including at least one line of array transducers that transmit and receive the ultrasonic signal to and from the subject.

In this regard, the transducers receives electric signals generated by the hybrid beamformer 200, converts the electric signals into ultrasonic signals, converts ultrasonic signals reflected from the subject into electrical signals, and transmits the electrical signals to the hybrid beamformer 200.

The probe 100 includes such transducers in a 2-dimensional (2D) array form, and, thus, the diagnosis system 10 may output the signal indicating the 3D diagnosis image. In this regard, although the diagnosis image may be the ultrasonic image, the diagnosis image is not limited thereto. The 2D array form means that M x N array transducers are arranged. M and N are integral numbers greater than or equal to 1, and may be the same number.

The each of the sub-arrays of the probe 100 include at least one line of array transducers. For example, if the probe 100 includes M × N transducers arranged in an elevation direction and a lateral direction, lines of array transducers may be arranged in the elevation direction or the lateral direction. In this regard, the sub-arrays may be array transducers including a line of transducers arranged in the elevation direction or array transducers including a line of transducers arranged in the lateral direction. However, the sub-arrays are not limited thereto, as the sub-arrays may be two or more lines of array transducers.

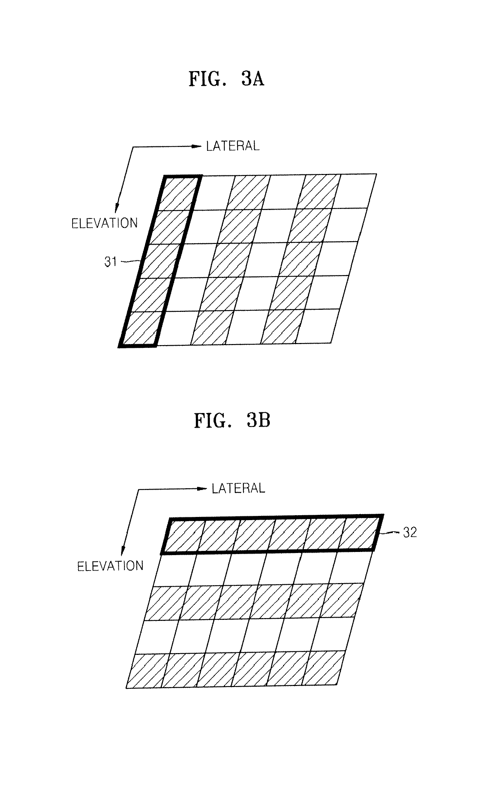

Accordingly, the hybrid beamformer 200 controls the transducers included in the probe 100 for each sub-array. This may reduce the number of cables required to control the transducers and an operation amount. In addition, the hybrid beamformer 200 may lessen a period of time required to generate a diagnosis image by reducing the operation amount. The sub-arrays will be described with reference to FIGS. 3A through 3D.

The signal generated by the hybrid beamformer 200 may be an electrical signal. The probe 100 may convert the electrical signal generated by the hybrid beamformer 200 into an ultrasonic signal. The probe 100 transmits the ultrasonic signal to the subject, and converts the ultrasonic signal reflected from the subject into an electrical signal. The hybrid beamformer 200 combines electrical signals converted by the probe 100, and forms a reception beam indicating information regarding the subject. Accordingly, the reception beam formed by the hybrid beamformer 200 may be generated as a diagnosis image regarding the subject according to predetermined processing such as digital signal processing (DSP), etc.

The control unit 210 controls a general operation of the hybrid beamformer 200 by controlling the signal generating unit 212, the transmission/reception switching unit 214, the reception signal processing unit 216, the analog beamformer 220, the ADC 230, and the digital beamformer 240. For example, the control unit 210 calculates time delay values according to distance differences between the transducers included in the probe 100 and a focal point of the subject, so as the transmission and reception beams to be formed according to the calculated time delay values, and transmission and reception signals to be generated according to the formed transmission and reception beams.

The control unit 210 of the hybrid beamformer 200 calculates time delay values for digital beamforming for each of the sub-arrays, and controls the transducers to transmit and receive the signals to and from the subject according to the time delay values calculated for each sub-array. Furthermore, the control unit 210 of the hybrid beamformer 200 calculates time delay values for analog beamforming for each of the transducers included in one of the sub-arrays, and controls the transducers to transmit and receive the signals to and from and the subject according to the time delay values calculated for each sub-array. In this regard, since the transducers, which are included in the sub-arrays and are disposed in the same position in a direction perpendicular to a direction in which the sub-arrays are arranged, have the same time delay values for analog beamforming, the control unit 210 calculates the time delay value for analog beamforming with respect to each of the transducers included in one of the sub-arrays. Furthermore, although the time delay values for analog beamforming may be calculated according to distances between the transducers and the focal point, the time delay values are not limited thereto.

A method of calculating time delay values for digital beamforming and a method of calculating time delay values for analog beamforming would be understood by one of ordinary skill in the art, and thus detailed descriptions thereof will be omitted here.

The control unit 210 controls the analog beamformer 220 to perform analog beamforming in the direction in which the sub-arrays are arranged, and the digital beamformer 240 to perform digital beamforming in a direction perpendicular to the direction in which the sub-arrays are arranged.

For example, if the probe 100 includes first through third sub-arrays each including five transducers (for example, transducers a, b, c, d, and e in the direction in which the sub-arrays are arranged), the control unit 210 calculates time delay values for digital beamforming with respect to each of the first through the third sub-arrays. If the calculated time delay values are d1, d2, and d3, the control unit 210 controls the transducers included in the first sub-array to transmit and receive the signals to and from the subject according to the time delay value d1, the transducers included in the second sub-array to transmit and receive the signals to and from the subject according to the time delay value d2, and the transducers included in the third sub-array to transmit and receive the signals to and from the subject according to the time delay value d3.

Furthermore, the control unit 210 calculates time delay values for analog beamforming with respect to the transducers a, b, c, d, and e included in one of the first through third sub-arrays. In this regard, the control unit 210 controls the transducers a, b, c, d, and e, which are disposed in the same position in the direction perpendicular to the direction in which the sub-arrays are arranged, to transmit and receive the signals to and from the subject according to the same time delay value.

For example, the control unit 210 calculates time delay values (for example, t1 through t5) for analog beamforming with respect to the transducers a, b, c, d, and e included in the first sub-arrays. In other words, the control unit 210 controls the transducer a included in the first sub-array, the transducer a included in the second sub-array, and the transducer a included in the third sub-array to transmit and receive signals to and from the subject according to the same time delay value t1 based on the calculated time delay values.

Although an example sub-array is described, the sub-arrays are not limited thereto. Time delay values for analog beamforming may be calculated with respect to another sub-array.

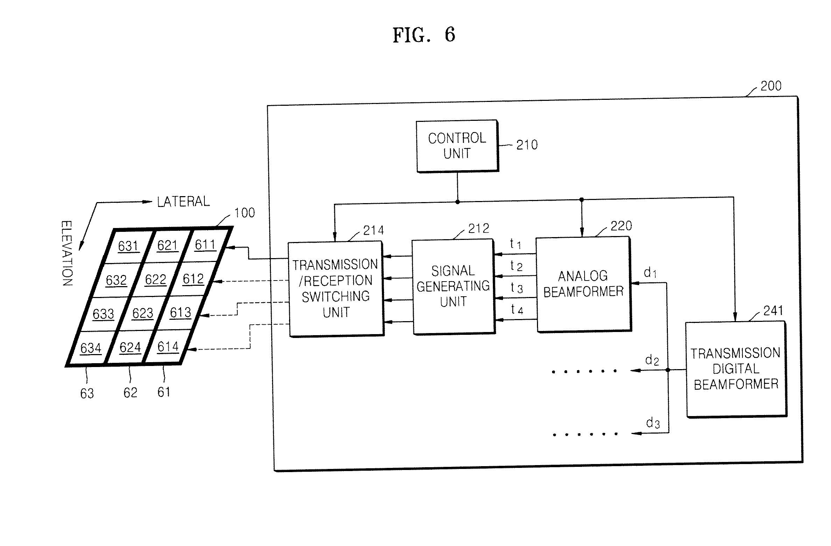

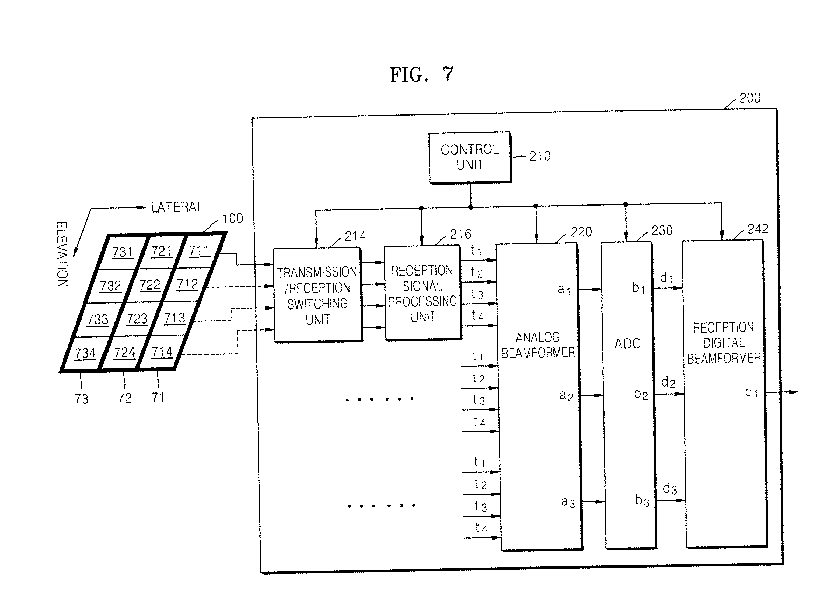

The control unit 210 of the hybrid beamformer 200 controls the transducers included in the probe 100 to have predetermined time delay values (for example, d1 through d3) for digital beamforming for each sub-array. The transducers that are included in each of the sub-arrays and are disposed in the same position in the direction perpendicular to the direction in which the sub-arrays are arranged to have predetermined time delay values (for example, t1 through t5) for analog beamforming. This will be described with reference to FIGS. 6 and 7.

However, the hybrid beamformer 200 is not limited thereto. It would be understood by one of ordinary skill in the art that the control unit 210 controls the transducers included in the probe 100 to transmit and receive signals between the subject and one or more of the five transducers included in the first sub-array according to the time delay value d1, to transmit and receive signals between the subject and one or more of the five transducers included in the second sub-array according to the time delay value d2, and to transmit and receive signals between the subject and one or more of the five transducers included in the third sub-array according to the time delay value d3.

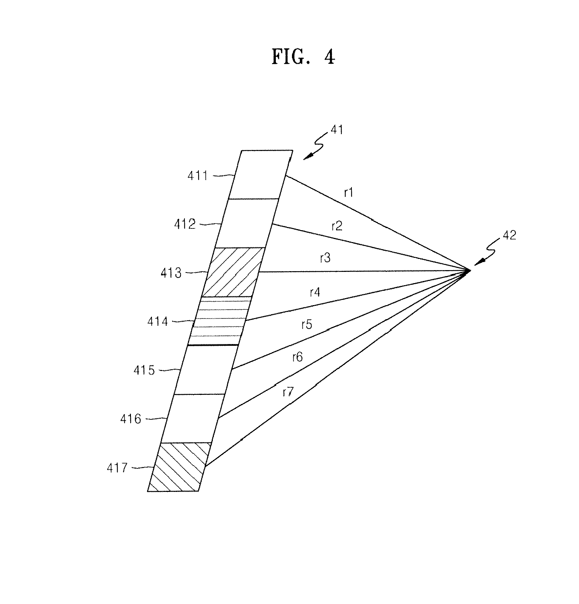

Regarding a method of calculating the time delay values d1 through d3 for digital beamforming for each sub-array, for example, the control unit 210 may calculate a time delay value with respect to a transducer closest to a focal point, calculate a time delay value with respect to a transducer farthest from the focal point, calculate a time delay value with respect to a transducer disposed in a center of the transducers, or calculate a time delay value by using an average of distances between the transducers and the focal point, from among the transducers included in a sub-array. However, the method of calculating the time delay values d1 through d3 for digital beamforming for each sub-array is not limited thereto. This will be described with reference to FIG. 4.

The control unit 210 of the hybrid beamformer 200 calculates time delay values for digital beamforming by using the same reference with respect to each of the sub-arrays. Thus, the sub-arrays may have different time delay values.

Accordingly, the control unit 210 controls the transducers included in the sub-arrays to have appropriate time delay values and transmit and receive signals with the subject, thereby improving definition of a diagnosis image generated by the reception beam formed by the hybrid beamformer 200.

When control lines are connected to the transducers included in the probe 100 to transmit and receive signals between the transducers and the subject according to time delay values for analog beamforming, the hybrid beamformer 200 of the diagnosis system 10 may use the same time delay value for analog beamforming with respect to the transducers that are included in the sub-arrays and are disposed in the same position in the direction perpendicular to the direction in which the sub-arrays are arranged. Further, the control lines may be connected to each sub-array. Thus, operation load may be remarkably reduced, time for generating a diagnosis image may be remarkably reduced, and resolution of the diagnosis image may be improved, compared to when the control lines are connected to all the transducers.

Furthermore, control lines necessary for analog beam focusing were determined according to the number of 2D plane images included in a 3D image. For example, when 256 2D plane images are necessary, 256 control signals for selection of a time delay element were necessary. However, since the hybrid beamformer 200 of the diagnosis system 10 uses the same time delay values for analog beamforming with respect to the transducers that are included in the sub-arrays and are disposed in the same position in the direction perpendicular to the direction in which the sub-arrays are arranged, control lines are connected to the respective sub-arrays, thereby reducing the number of control lines. For example, if 256 2D plane images are generated in eight sub-arrays, only eight control signals may be used.

The signal generating unit 212 generates a transmission signal by using the transmission beam formed by the analog beamformer 220. For example, the signal generating unit 212 generates a transmission pulse that is to be transmitted to the subject through the probe 100. In addition, the signal generating unit 212 of the hybrid beamformer 200 may be an ultrasonic transmission pulser that generates an ultrasonic transmission pulse. However, the signal generating unit 212 is not limited thereto.

The transmission/reception switching unit 214 performs a switching operation to transmit and receive signals from and to the transducers of each sub-array with respect to a signal generated by the ultrasonic transmission pulser, a signal received by the probe 100, or a combination thereof. For example, transmission and reception of signals for each sub-array may be operations performed according to each channel.

The reception signal processing unit 216 performs a predetermined processing operation with respect to the signal received by the probe 100. For example, the reception signal processing unit 216 may include a low noise amplifier (LNA) (not shown) that reduces noise of an analog signal received from the probe 100, and a variable gain amplifier (VGA) (not shown) that controls a gain value according to an input signal. In this regard, the VGA may be a time gain compensator (TGC) that compensates for a gain according to a distance between a focal point and the VGA. However, the reception signal processing unit 216 is not limited thereto.

The signal generating unit 212, the transmission/reception switching unit 214, and the reception signal processing unit 216 would be understood by one of ordinary skill in the art, and thus detailed descriptions thereof will be omitted here.

The analog beamformer 220 forms a transmission beam under control of the control unit 210, outputs the transmission beam to the signal generating unit 212, combines signals received by the transducers included in the sub-arrays according to time delay values for analog beamforming, and generates analog signals with respect to each of the sub-arrays. That is, the analog beamformer 220 of the hybrid beamformer 200 performs fixed focusing and analog beamforming.

For example, the analog beamformer 220 performs analog beamforming according to time delay values for analog beamforming according to distances between the focal point and each of the transducers included in one of the sub-arrays. In this regard, the transducers that are included in the sub-arrays and are disposed in the same position in the direction perpendicular to the direction in which the sub-arrays are arranged have the same time delay values for analog beamforming.

In this regard, although one of the sub-arrays may be the closest to the focal point, the sub-arrays are not limited thereto. One of the sub-arrays may be the farthest from the focal point, may be disposed in the center of the sub-arrays, or various other cases may exist.

Furthermore, the analog beamformer 220 of the hybrid beamformer 200 may steer a signal transmitted from the probe 100 to the subject in the direction in which the sub-arrays are arranged.

For example, if the sub-arrays include a line of all transducers arranged in an elevation direction, the analog beamformer 220 steers signals transmitted from the transducers of the probe 100 to the subject only in the elevation direction.

For another example, if the sub-arrays include a line of all transducers arranged in a lateral direction, the analog beamformer 220 steers signals transmitted from the transducers of the probe 100 to the subject only in the lateral direction.

In this manner, the analog beamformer 220 steers signals transmitted from the probe 100 to the subject in the direction in which the sub-arrays are arranged, thereby improving resolution of a diagnosis image that may be generated by the reception beam formed by the hybrid beamformer 200. For example, if the sub-arrays include a line of all transducers arranged in the elevation direction, since the time delay values for digital beamforming calculated for each sub-array in the control unit 210 does not consider distances between the focal point and each of the transducers included in the sub-arrays, the analog beamformer 220 steers signals transmitted from the transducers to the subject in the direction in which the sub-arrays are arranged.

Accordingly, the analog beamformer 220 may improve resolution of a generated diagnosis image while not increasing operation load. In addition, it would be understood by one of ordinary skill in the art that the analog beamformer 220 may steer the transducers in a direction other than the direction in which the sub-arrays are arranged so as to improve the resolution of the diagnosis.

A method of performing analog beamforming in the analog beamformer 220 would be understood by one of ordinary skill in the art, and thus a detailed description thereof will be omitted here.

As described above, the analog beamformer 220 of the hybrid beamformer 200 may generate analog signals with respect to the sub-arrays. If signals transmitted from the probe 100 are steered and are reflected from the subject, the analog beamformer 220 combines the reflected signals received by the transducers, and generates analog signals for each of the sub-arrays.

The ADC 230 converts each of the analog signals generated by the analog beamformer 220 into a digital signal. Thus, the ADC 230 generates digital signals, and outputs the digital signals to the digital beamformer 240.

The digital beamformer 240 forms a transmission beam under control of the control unit 210, outputs the transmission beam to the analog beamformer 220, and combines the digital signals converted by the ADC 230 according to time delay values for digital beamforming calculated for each of the sub-arrays.

For example, the transmission digital beamformer 241 forms a transmission beam under control of the control unit 210, and outputs the transmission beam to the analog beamformer 220.

The reception digital beamformer 242 combines the digital signals converted by the ADC 230 according to time delay values for digital beamforming calculated for each of the sub-arrays, and forms a reception beam.

For example, the analog beamformer 220 combines the signals received by the array transducers included in the sub-arrays according to the time delay values for analog beamforming, and generates the analog signals with respect to the sub-arrays. The ADC 230 converts the analog signals generated by the analog beamformer 220 into digital signals, and the reception digital beamformer 242 combines the digital signals converted by the ADC 230 according to the time delay values for digital beamforming calculated by the control unit 210.

In this manner, the digital beamformer 240 performs dynamic focusing to form the transmission and reception beams in consideration of the time delay values for digital beamforming calculated for each of the sub-arrays. For example, the reception digital beamformer 242 of the digital beamformer 240 performs dynamic focusing with respect to the analog signals generated by the analog beamformer 220, thereby dramatically reducing the number of cables connected to the digital beamformer 240.

A method of performing digital beamforming in the digital beamformer 240 would be understood by one of ordinary skill in the art, and thus a detailed description thereof will be omitted here.

Therefore, the hybrid beamformer 200 may form a reception beam capable of generating high definition diagnosis images while reducing the number of cables, the number of control lines, and operation load.

FIGS. 3A through 3D illustrate examples of sub-arrays of the probe 100 of the diagnosis system 10.

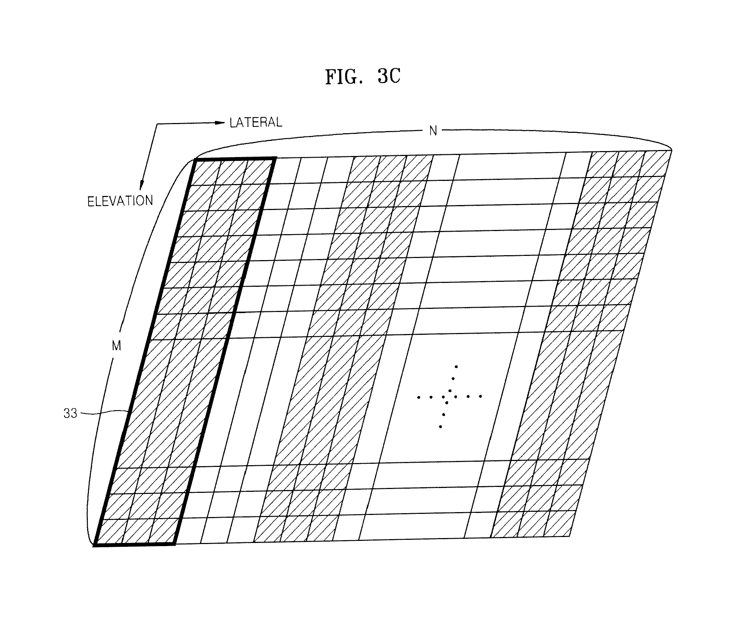

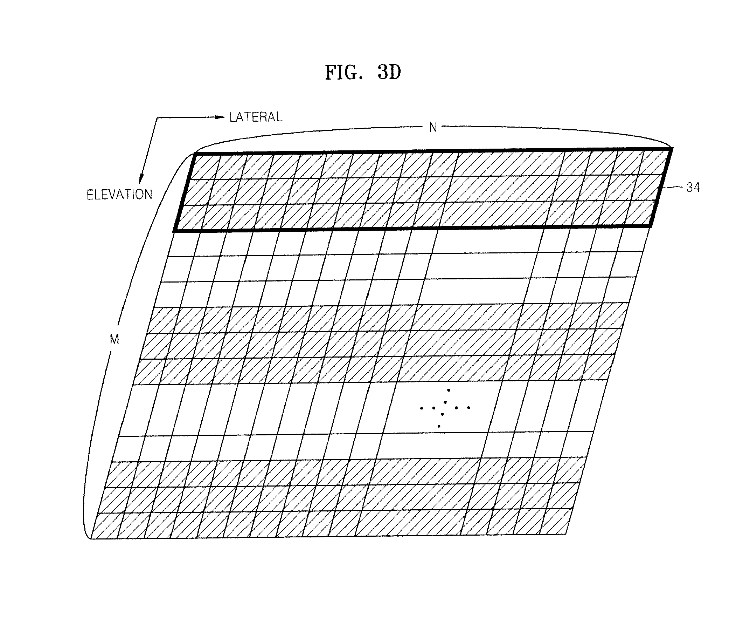

For descriptive convenience, although FIGS. 3A and 3B illustrate 5x6 transducers arranged in elevation and lateral directions, and FIGS. 3C and 3D illustrate M × N transducers arranged in the elevation and lateral directions, the sub-arrays are not limited thereto.

Referring to FIGS. 2 and 3A, the probe 100 may include a sub-array 31 that includes an array transducer including a line of all transducers arranged in the elevation direction. Thus, the probe 100 may include six sub-arrays.

Referring to FIGS. 2 and 3B, the probe 100 may include a sub-array 32 that includes an array transducer including a line of all transducers arranged in the lateral direction. Thus, the probe 100 may include five sub-arrays.

Referring to FIGS. 2 and 3C, the probe 100 may include a sub-array 33 that includes array transducers including three lines of all transducers arranged in the elevation direction. In this regard, the sub-array 33 is not limited to the three lines of transducers, and may include two lines of transducers, four lines of transducers, or the like.

Referring to FIGS. 2 and 3D, the probe 100 may include a sub-array 34 that includes array transducers including three lines of all transducers arranged in the lateral direction. In this regard, the sub-array 34 is not limited thereto, and may include two lines of transducers, four lines of transducers, and the like.

Accordingly, the hybrid beamformer 200 controls the transducers included in the probe 100 for each sub-array, thereby reducing the number of cables used to control the transducers and operation load.

Furthermore, the probe 100 of the diagnosis system 10 may include a line of array transducers as a sub-array so as to obtain a high resolution 3D diagnosis image, and two or more array transducers as a sub-array so as to obtain a low resolution 3D diagnosis image by adaptively adjusting the number of array transducers included in a sub-array. Thus, the sub-array may be appropriately adjusted according to a user environment, thereby controlling trade-off between operation load and the resolution of a diagnosis image according to user convenience.

FIG 4 illustrates an example of a method of calculating time delay values for digital beamforming for each sub-array in the control unit 210 of the hybrid beamformer 200 of the diagnosis system 10. FIG 4 illustrates a sub-array 41 including a line of array transducers and a focal point 42 of a subject.

For example, the control unit 210 may calculate a time delay value for a sub-array 41 with respect a transducer 413 closest to the focal point 42 from among transducers included in the sub-array 41.

As another example, the control unit 210 may calculate a time delay value for the sub-array 41 with respect a transducer 417 farthest from the focal point 42 from among the transducers included in the sub-array 41.

As another example, the control unit 210 may calculate a time delay value for the sub-array 41 with respect a transducer 414 disposed in the center of the transducers included in the sub-array 41.

As another example, the control unit 210 may calculate a time delay value for the sub-array 41 by using an average of distances between the focal point 42 and the transducers included in the sub-array 41. For example, the control unit 210 may calculate the time delay value for the sub-array 41 by using an average of distances r1 through r7 between the focal point 42 and the transducers included in the sub-array 41.

In this regard, the control unit 210 may calculate a time delay value for each of the sub-arrays by using the same reference, thereby calculating the time delay value for each of the sub-arrays.

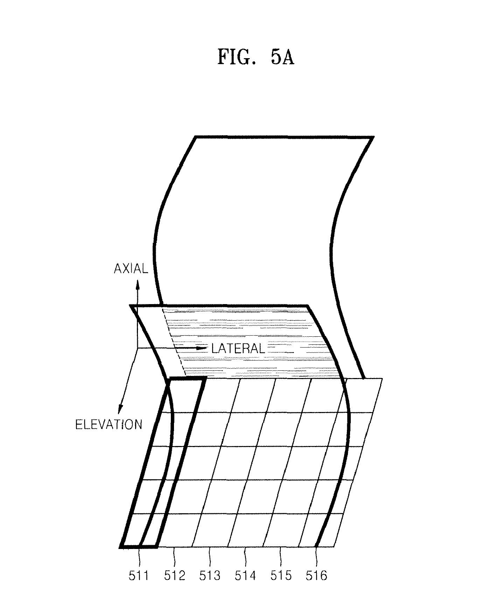

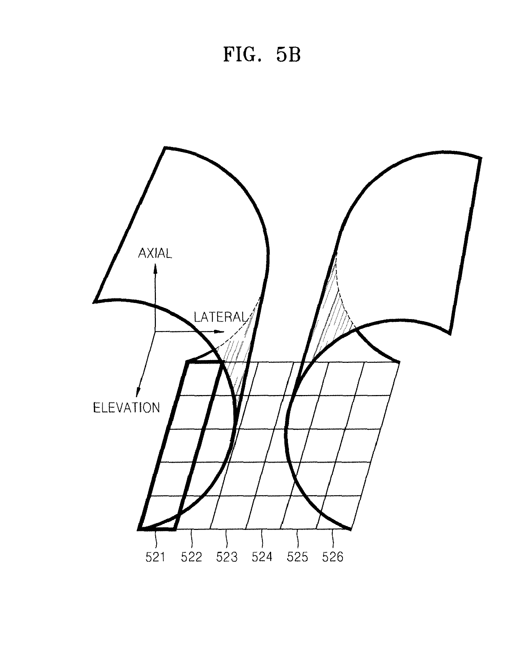

FIGS. 5A and 5B illustrate elevation and lateral directions of sub-arrays of the probe 100 of the diagnosis system 10. For descriptive convenience, although a sub-array includes an array transducer including a line of all transducers arranged in the elevation direction, the sub-arrays are not limited thereto.

Referring to FIG. 5A, transmission and reception beam focusing is performed in the elevation direction. Referring to FIG. 5B, the transmission and reception beam focusing is performed in the lateral direction.

The transmission and reception beam focusing will now be described with reference to FIGS. 2 and 5A. The probe 100 and the analog beamformer 220 perform the transmission and reception beam focusing in the elevation direction by using an analog element. In this regard, each of six sub-arrays 511 through 516 is connected to a control line. The control unit 210 may determine a beam focusing position with respect to the elevation direction of the analog beamformer 220, through the control lines. In this regard, the control unit 210 controls transducers that are included in the sub-arrays 511 through 516 and are disposed in the same position in a direction perpendicular to a direction in which each of the sub-arrays 511 through 516 is arranged to transmit and receive signals with a subject according to the same time delay value for analog beamforming.

That is, the control unit 210 determines a signal transmission and reception direction and a beam focusing position in the elevation direction, and the analog beamformer 220 adjusts signals according to the determined signal transmission and reception direction and the beam focusing position.

Performing transmission and reception beam focusing by using the analog element is similar to using a sound lens in the elevation direction in a 1-dimensional (1D) array transducer. However, the 1D array transducer mechanically moves to determine the signal transmission and reception direction in the elevation direction. That is, although a 3D diagnosis image may be obtained by mechanically moving the 1D array transducer, such a method has limited performance in terms of an image forming speed, i.e. in terms of temporal resolution or spatial resolution.

For example, since the 1D array transducer performs fixed focusing by using the sound lens in the elevation direction, an image has the highest spatial resolution near a focal point in the elevation direction and low spatial resolution in points other than the focal point. Also, although the 3D diagnosis image may be obtained by mechanically moving the 1D array transducer, since the 1D array transducer mechanically moves at a limited speed, temporal resolution is reduced to one frame per two seconds.

Meanwhile, the analog beamformer 220 of the hybrid beamformer 200 electrically determines the signal transmission and reception direction by using 2D array transducers, and, thus, the performance of temporal resolution and spatial resolution may be guaranteed.

For example, the hybrid beamformer 200 of the diagnosis system 10 improves temporal resolution and spatial resolution by using 2D array transducers, thereby obtaining high resolution volumetric images and multi-plane images in real-time. Furthermore, the hybrid beamformer 200 may reduce the number of cables by using sub-arrays while using 2D array transducers.

Therefore, the analog beamformer 220 determines a signal transmission and reception direction and a beam focusing position in the elevation direction, and the digital beamformer 240 performs transmission and reception beam focusing in the lateral direction.

In this regard, the digital beamformer 240 is connected to signal transmission cables that may be connected to the ADC 230. The control unit 210 controls the digital beamformer 240 to transmit and receive signals according to time delay values for digital beamforming calculated for each sub-array through the signal transmission cables.

Performing transmission and reception beam focusing by using a digital element is similar to obtaining a 2D plane image by using 1 D array transducers.

Therefore, the diagnosis system 10 performs analog beamforming in the elevation direction that is a direction in which the sub-arrays are arranged, and performs digital beamforming in the lateral direction. Thus, the diagnosis system 10 may generate a signal for generating a high definition 3D diagnosis image while reducing the number of control lines connected to the analog beamformer 220, the number of cables connected to the digital beamformer 240, and operation load.

As described above, although a sub-array includes an array transducer including a line of all transducers arranged in the elevation direction in FIGS. 5A and 5B, the sub-arrays are not limited thereto. It would be understood by one of ordinary skill in the art that a sub-array includes an array transducer including a line of all transducers arranged in the lateral direction.

For example, the diagnosis system 10 may perform analog beamforming in the lateral direction that is a direction in which the sub-arrays are arranged, and performs digital beamforming in the elevation direction.

FIG 6 illustrates a transmission beamforming operation of the diagnosis system 10 . For descriptive convenience, although a sub-array includes an array transducer including a line of all transducers arranged in an elevation direction, the transmission beamforming operation is not limited thereto.

Referring to FIGS. 2 and 6, the probe 100 includes three sub-arrays 61, 62, and 63 that respectively include transducers 611 through 614, 621 through 624, and 631 through 634.

The control unit 210 calculates time delay values for digital beamforming with respect to each of the sub-arrays 61, 62, and 63 and analog beamforming with respect to each of transducers 611 through 614, 621 through 624, and 631 through 634 included in one of the sub-arrays 61, 62, and 63. The control unit 210 controls the transducers 611 through 614, 621 through 624, and 631 through 634 to transmit and receive signals to and from a subject according to the calculated time delay values.

Accordingly, the transmission digital beamformer 241 applies the time delay values d1, d2, and d3 for digital beamforming to the transducers 611 through 614, 621 through 624, and 631 through 634 included in the first through third sub-arrays 61, 62, and 63, respectively, and the analog beamformer 220 applies the time delay values t1, t2, t3, and t4 for analog beamforming to each of the transducers 611 through 614, 621 through 624, and 631 through 634 included in the first through third sub-arrays 61, 62, and 63, respectively.

Regarding the transducers 611 through 614 included in the first sub-array 61, the transducer 611 transmits a signal to the subject according to a time delay value to which the time delay value d1 for digital beamforming and the time delay value t1 for analog beamforming are applied. The transducer 612 transmits a signal to the subject according to a time delay value to which the time delay value d1 for digital beamforming and the time delay value t2 for analog beamforming are applied. The transducer 613 transmits a signal to the subject according to a time delay value to which the time delay value d1 for digital beamforming and the time delay value t3 for analog beamforming are applied. The transducer 614 transmits a signal to the subject according to a time delay value to which the time delay value d1 for digital beamforming and the time delay value t4 for analog beamforming are applied.

Regarding the transducers 621 through 624 included in the sub-array 62, the transducer 621 transmits a signal to the subject according to a time delay value to which the time delay value d2 for digital beamforming and the time delay value t1 for analog beamforming are applied. In this way, time delay values for the transducers 621 through 624 and 631 through 634 included in the second sub-array 62 and the third sub-array 63, respectively, may be determined.

The transducers 611**,** 621, and 631 disposed in the same position in a lateral direction that is a direction perpendicular to a direction in which the sub-arrays 61, 62, and 63 are arranged have the same time delay value t1 for analog beamforming.

The signal generating unit 212 generates an electrical signal according to the transmission beam formed by the analog beamformer 220. The transmission and reception switching unit 214 performs switching to transmit signals for each of the sub-arrays 61, 62, and 63.

Accordingly, the diagnosis system 10 may perform transmission beam focusing to obtain a high definition diagnosis image while reducing the numbers of control lines and cables.

FIG 7 illustrates an example of a reception beamforming operation of the diagnosis system 10. For descriptive convenience, although a sub-array includes an array transducer including a line of all transducers arranged in an elevation direction, the sub-arrays are not limited thereto.

Referring to FIGS. 2 and 7, the probe 100 includes three sub-arrays 71, 72, and 73 that respectively include transducers 711 through 714, 721 through 724, and 731 through 734.

The transmission and reception switching unit 214 performs switching to receive signals for each of the sub-arrays 71, 72, and 73. The reception signal processing unit 216 performs predetermined processing, such as noise reduction, gain amplification, etc., on the received signals.

The analog beamformer 220 generates an analog signal a1 by combining the signals that are received by the transducers 711 through 714 included in the sub-array 71 and processed by the reception signal processing unit 216 according to the delay signal values t1, t2, t3, and t4 for analog beamforming used to form the transmission beam. Thus, the analog beamformer 220 generates three analog signals a1, a2, and a3 by combining the signals received by the transducers 711 through 714, 721 through 724, and 731 through 734 included in the sub-arrays 71, 72, and 73, respectively.

The ADC 230 converts the three analog signals a1, a2, and a3 into three digital signals b1, b2, and b3.

The reception digital beamformer 242 generates one or more digital signals c1 by combining the three digital signals b1, b2, and b3 according to the time delay values d1 through d3 for digital beamforming calculated for each sub-array. The one or more digital signals c1 generated by the reception digital beamformer 242 may indicate information regarding a focal point of a subject or the subject that is to be an image, and may be used to generate a diagnosis image.

When the probe 100 includes 4 x 3 transducers arranged in elevation and lateral directions, 12 control lines or cables are necessary for performing analog beamforming or digital beamforming.

The diagnosis system 10 may perform beamforming by using 4 control lines and 3 cables when the sub-arrays are arranged in the elevation direction. Thus, the diagnosis system 10 reduces the number of control lines and cables, thereby generating a signal used to generate a high definition 3D diagnosis image, and dramatically reducing operation load.

As described above, although a sub-array includes an array transducer including a line of all transducers arranged in the elevation direction in FIGS. 6 and 7, the sub-arrays are not limited thereto. It would be understood by one of ordinary skill in the art that a sub-array may include an array transducer including a line of all transducers arranged in the lateral direction. For example, the diagnosis system 10 may perform beamforming by using only 3 control lines and 4 cables.

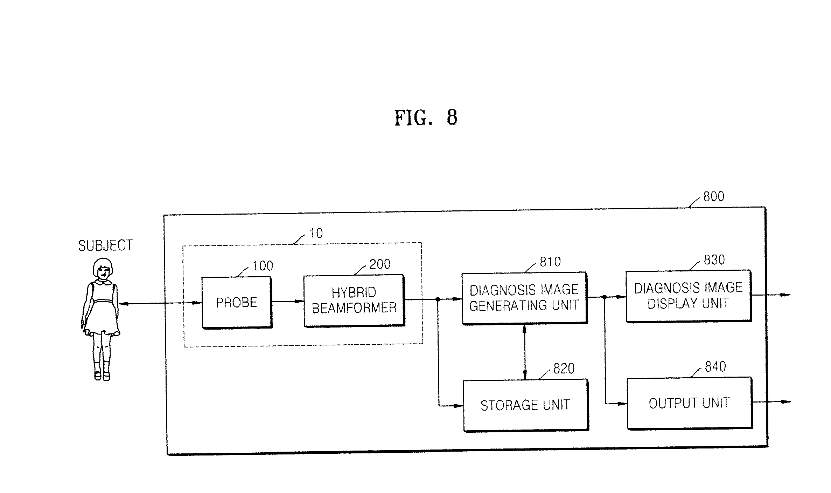

FIG. 8 is a block diagram illustrating an example of a medical image system 800. Referring to FIG 8, the medical image system 800 includes the diagnosis system 10, a diagnosis image generating unit 810, a storage unit 820, a diagnosis image display unit 830, and an output unit 840. The diagnosis system 10 includes the probe 100 and the hybrid beamformer 200.

FIG. 8 illustrates the elements of the medical image system 800. Accordingly, it would be understood by one of ordinary skill in the art that the medical image system 800 may further include general-purpose elements other than the elements illustrated in FIG. 8.

Furthermore, the descriptions with reference to the diagnosis system 10 of FIGS. 1 and 2 apply to the medical image system 800 of FIG 8, and, thus, detailed descriptions thereof will be omitted here.

The medical image system 800 provides a diagnosis image regarding a subject. For example, the medical image system 800 displays the diagnosis image regarding the subject or outputs a signal indicating the diagnosis image regarding the subject to an external device that displays the diagnosis image regarding the subject.

Thus, the diagnosis system 10 outputs a signal used to generate the diagnosis image regarding the subject by using the probe 100 and the hybrid beamformer 200.

The probe 100 includes sub-arrays each including at least one line of array transducers that transmit and receive signals to and from the subject. The hybrid beamformer 200 forms a reception beam by performing analog beamforming in a direction in which the sub-arrays are arranged, and performing digital beamforming in a direction perpendicular to the direction in which the sub-arrays are arranged.

In this regard, the direction in which the sub-arrays are arranged may be an elevation direction or a lateral direction, and, thus, the direction perpendicular to the direction in which the sub-arrays are arranged may be the lateral direction or the elevation direction.

Furthermore, the hybrid beamformer 200 of the diagnosis system 10 includes an analog beamformer that performs analog beamforming. The analog beamformer steers the signal transmitted from the probe 100 to the subject in the direction in which the sub-arrays are arranged.

The hybrid beamformer 200 of the diagnosis system 10 includes a control unit that calculates time delay values for digital beamforming for each of the sub-arrays, controls the transducers to transmit and receive signals to and from the subject according to the time delay values for digital beamforming calculated for each of the sub-arrays, calculates time delay values for analog beamforming for each of the transducers included in one of the sub-arrays, and controls the transducers, that are included in the sub-arrays and are disposed in the same position in the direction perpendicular to the direction in which the sub-arrays are arranged, to transmit and receive signals to and from the subject according to the same time delay value for analog beamforming.

Accordingly, the hybrid beamformer 200 of the diagnosis system 10 may form a reception beam used to generate a high definition 3D diagnosis image and reduce operation load.

The diagnosis image generating unit 810 generates the diagnosis image by using the reception beam formed by the hybrid beamformer 200. For example, the diagnosis image generating unit 810 may include a digital signal processor (DSP) (not shown) and a digital scan converter (DSC) (not shown). The DSP of the diagnosis image generating unit 810 forms image data that indicates b, c, or d mode by processing a signal output from the hybrid beamformer 200, a signal stored in the storage unit 820, or a combination thereof. The DSC of the diagnosis image generating unit 810 generates a scanned diagnosis image to display the image data formed by the DSP.

The DSP and the DSC would be understood by one of ordinary skill in the art, and thus detailed descriptions thereof will be omitted here.

The storage unit 820 stores data generated during an operation of the medical image system 800. For example, the storage unit 820 may store the reception beam formed by the hybrid beamformer 200 or may store the image data indicating the b, c, or d mode or the scanned diagnosis image.

The storage unit 820 of the medical image system 800 is a general storage medium, and it would be understood by one of ordinary skill in the art that the storage unit 820 of the medical image system 800 may include a hard disk drive (HDD), a rean only memory (ROM), a random access memory (RAM), a flash memory, and a memory card.

The diagnosis image display unit 830 displays the diagnosis image generated by the diagnosis image generating unit 810. For example, the diagnosis image display unit 830 includes all output devices provided to the medical image system 800, such as a display panel, a mouse, a LCD screen, a monitor, etc.

However, it would be understood by one of ordinary skill in the art that the medical image system 800 may not include the diagnosis image display unit 830 and may include the output unit 840 for outputting the diagnosis image generated by the diagnosis image generating unit 810 to an external display device (not shown).

In this regard, the output unit 840 may transmit and receive data to and from an external device over wired and wireless networks or through wired serial communication. The networks include Internet, a local area network (LAN), a wireless LAN, a wide area network (WAN), a personal area network (PAN), etc. However, the output unit 840 is not limited thereto, and the networks may include different types of networks.

Therefore, it would be understood by one of ordinary skill in the art that the storage unit 820 and the output unit 840 of the medical image system 800 may be integrally formed in a picture archiving communication system (PACS) formed by further including image reading and retrieval functions.

Accordingly, the medical image system 800 may provide a user with a high definition 3D diagnosis image and reduce operation load.

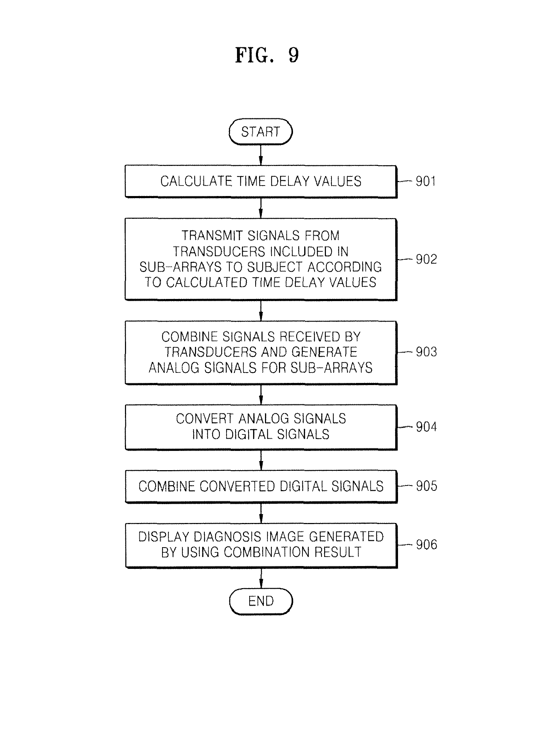

FIG 9 is a flowchart illustrating an example of a method of displaying a diagnosis image. The method of displaying the diagnosis image described with respect to FIG. 9 may include operations performed by the diagnosis system 10 or the medical image system 800 of FIGS. 1, 2, and 8. The method of displaying the diagnosis image described with respect to FIG**.** 9 may also include those operations being performed sequentially. Thus, although not provided below, the descriptions of the diagnosis system 10 or the medical image system 800 of FIGS. 1, 2, and 8 may be applied to the method described with respect to FIG. 9.

In operation 901, the control unit 210 calculates time delay values for digital beamforming for each of a plurality of sub-arrays included in the probe 100 and time delay values for analog beamforming for each of the transducers included in one of the sub-arrays. In this regard, the sub-arrays include at least one line of array transducers, and the transducers disposed in the same position in a direction perpendicular to a direction in which the sub-arrays are arranged have the same time delay value for analog beamforming.

In operation 902, the probe 100 transmits signals to a subject according to time delay values calculated in operation 901. For example, the transducers included in the sub-arrays of the probe 100 transmit signals to the subject according to the time delay values for digital beamforming and the time delay values for analog beamforming. The signals transmitted from the probe 100 to the subject are steered in the direction in which the sub-arrays are arranged under control of the hybrid beamformer 200.

In operation 903, the analog beamformer 220 combines the signals received by the transducers included in the sub-arrays according to the time delay values for analog beamforming calculated in operation 901, and generates a plurality of analog signals for the sub-arrays.

In operation 904, the ADC 230 converts the analog signals into digital signals.

In operation 905, the digital beamformer 240 combines the converted digital signals according to the time delay values for digital beamforming calculated in operation 901.

In operation 906, the diagnosis image display unit 830 displays a diagnosis image generated by using a result of combining the converted digital signals. In this regard, the diagnosis image may be generated by the diagnosis image generating unit 810.

Accordingly, the method of displaying the diagnosis image may display a high definition 3D diagnosis image while reducing operation load.

According to the diagnosis system 10 and the medical image system 800, 2D array transducers are used to obtain optimal spatial resolution in all 3D image points, and an electrical switching method other than a mechanical movement method is used to obtain a high definition 3D diagnosis image in real-time. The real-time high definition 3D diagnosis image is provided in such a way that a user may easily recognize anatomic information regarding human organs, thereby increasing an exact diagnosis of a disease and diagnosis convenience. Furthermore, new clinical information such as a 3D color flow image of a heart may be provided to a medical expert.

In addition, a medical image apparatus may have difficulty realizing beam focus depending to the hardware, which may directly affect image quality. Nevertheless, the diagnosis system 10 and the medical image system 800 may reduce the number of cables while using 2D array transducers, thereby reducing hardware complexity and maximizing system efficiency.

According to teachings provided above, there is provided a hybrid beamformer that may reduce complexity and operation load of hardware used to generate and display a 3D diagnosis image of a subject. Accordingly, the diagnosis system 10 and the medical image system 800 and the method of displaying a diagnosis image may realize synthetic aperture focusing techniques by minimizing complexity of the hardware.

Program instructions to perform a method described herein, or one or more operations thereof, may be recorded, stored, or fixed in one or more computer-readable storage media. The program instructions may be implemented by a computer. For example, the computer may cause a processor to execute the program instructions. The media may include, alone or in combination with the program instructions, data files, data structures, and the like. Examples of computer-readable media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The program instructions, that is, software, may be distributed over network coupled computer systems so that the software is stored and executed in a distributed fashion. For example, the software and data may be stored by one or more computer readable recording mediums. Also, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein. Also, the described unit to perform an operation or a method may be hardware, software, or some combination of hardware and software. For example, the unit may be a software package running on a computer or the computer on which that software is running.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A diagnosis system (10), comprising:
a probe (100) comprising a plurality of sub-arrays, each of the sub-arrays comprising at least one line of array transducers, the array transducers being configured to transmit and receive signals to and from a subject being diagnosed; and
a hybrid beamformer (200) configured to:
perform analog beamforming in a direction in which the sub-arrays are arranged; and
perform digital beamforming in a direction perpendicular to the direction in which the sub-arrays are arranged.

2. The diagnosis system of claim 1, wherein the hybrid beamformer comprises:
a control unit (210) configured to:
calculate time delay values for the digital beamforming for each of the sub-arrays; and
control the transducers included in each of the sub-arrays to transmit and receive the signals to and from the subject according to the time delay values calculated for each of the sub-arrays.

3. The diagnosis system of claim 2, wherein the control unit is further configured to:
calculate time delay values for the analog beamforming for each of transducers included in one of the sub-arrays, wherein the calculated time delay values for transducers being disposed in the same position in the direction perpendicular to the direction in which the sub-arrays are arranged, are the same; and
control the transducers to transmit and receive the signals to and from the subject according to the calculated time delay values for the analog beamforming.

4. The diagnosis system of one claims 1 to 3, wherein the hybrid beamformer comprises:
an analog beamformer (220) configured to:
combine the signals received by the transducers included in the sub-arrays according to the time delay values for the analog beamforming; and
generate a plurality of analog signals for each of the sub-arrays.

5. The diagnosis system of claim 4, wherein the analog beamformer is further configured to steer the signals transmitted from the probe to the subject in the direction in which the sub-arrays are arranged.

6. The diagnosis system of claim 2 or 3, wherein the hybrid beamformer further comprises:
an analog beamformer (220) configured to:
combine the signals received by the transducers included in the sub-arrays according to the time delay values for analog beamforming; and
generate a plurality of analog signals for each of the sub-arrays;
an analog-digital converter configured to convert the generated analog signals into digital signals; and
a digital beamformer (240) configured to combine the converted digital signals according to the time delay values for the digital beamforming calculated for each of the sub-arrays.

7. The diagnosis system of claim 1, wherein the hybrid beamformer comprises:
an analog beamformer (220) configured to steer the signals transmitted from the transducers to the subject in an elevation direction when each of the sub-arrays includes a line of all transducers arranged in the elevation direction, or
an analog beamformer (220) configured to steer the signals transmitted from the transducers to the subject in a lateral direction when each of the sub-arrays includes a line of all transducers arranged in the lateral direction.

8. A medical image system (800), comprising:
a diagnosis system according to any one of claims 1 to 7;
wherein the hybrid beamformer configured to form a reception beam by performing the analog beamforming and performing the digital beamforming; and
the system further comprising a diagnosis image generating unit (810) configured to generate a diagnosis image for the subject by using the formed reception beam.

9. The medical image system of claim 8, wherein the hybrid beamformer comprises:
an analog beamformer (220) configured to:
perform the analog beamforming; and
steer signals transmitted from the probe to the subject in the direction in which the sub-arrays are arranged.

10. The medical image system of claim 8 or 9, further comprising:
a diagnosis image display unit (830) configured to display the generated diagnosis image.

11. A method of displaying a diagnosis image, the method comprising:
calculating time delay values for digital beamforming for each of a plurality of sub-arrays included in a probe and time delay values for analog beamforming for each of a plurality of transducers included in one of the sub-arrays;
transmitting signals from the transducers included in the sub-arrays to a subject according to the calculated time delay values;
combining the signals received by the transducers included in the sub-arrays according to the calculated time delay values for the analog beamforming;
generating a plurality of analog signals for each of the sub-arrays;
converting the analog signals into a plurality of digital signals;
combining the digital signals according to the time delay values for the digital beamforming;
and
displaying a diagnosis image generated by using a result of the combining of the digital signals,
wherein each of the sub-arrays comprises at least one line of the array transducers.

12. The method of claim 11, wherein the transmitting of the signals comprises:
steering the signals in a direction in which the sub-arrays are arranged.

13. The method of claim 11, wherein the transmitting of the signals comprises:
steering the signals in an elevation direction when each of the sub-arrays comprises a line of all transducers arranged in the elevation direction, or
steering the signals in a lateral direction when each of the sub-arrays comprises a line of all transducers arranged in the lateral direction.

14. The method of one of claims 11 to 13, wherein the transducers have the same time delay value calculated for the analog beamforming, the transducers being included in one of the sub-arrays and disposed in the same position in the direction perpendicular to the direction in which the sub-arrays are arranged.

15. A non-transitory computer-readable recording medium having stored thereon instructions, wherein execution of the instructions by one or more processors of a computer system causes the one or more processors to perform the method of one of claims 11 to 14.
